# EUROPEAN PATENT SPECIFICATION

(11) **EP 2 242 736 B1**
(45) Date of publication and mention of the grant of the patent: **14.09.2011**
(21) Application number: 08853527.3
(22) Date of filing: 27.11.2008
(51) Int. Cl.: C07C 45/63, C07D 495/04, C07C 49/567

(54) **PROCESS FOR THE PREPARATION OF PHARMACEUTICAL INTERMEDIATES**
VERFAHREN ZUR HERSTELLUNG VON PHARMAZEUTISCHEN ZWISCHENPRODUKTEN
PROCÉDÉ POUR LA PRÉPARATION D'INTERMÉDIAIRES PHARMACEUTIQUES

(30) Priority: 27.11.2007 HU 0700757
(43) Date of publication of application: 27.10.2010
(73) Proprietor: Egis Gyógyszergyár Nyilvánosan Müködö Részvénytársaság, 1106 Budapest (HU)
(72) Inventor: MEZEI, Tibor, H-1221 Budapest (HU); LUKÁCS, Gyula, H-1163 Budapest (HU); MOLNÁR, Enikó, H-2030 Erd (HU); BARKÓCZY, Jószef, H-1016 Budapest (HU); VOLK, Balázs, H-1165 Budapest (HU); PORCS-MAKKAY, Márta, H-2013 Pomáz (HU); SZULÁGYI, János, H-1033 Budapest (HU); VAJJON, Mária, H-Budapest 1136 (HU)
(74) Representative: Beszédes, Stephan G.
(86) International application number: PCT/HU2008/000139
(87) International publication number: WO 2009/068924

(56) References cited:
- WO-A-2007/114526
- US-A1- 2003 134 872

## Description

The invention relates to a process for the preparation of 2-halogen-1-cyclopropyl-2-substituted phenylethanones of formula (III) by the halogenation of 1-cyclopropyl-2-substituted phenylethanones of general formula (II) wherein the halogenation is carried out in the mixture of aqueous hydrogen halide and aqueous hydrogen peroxide in the present of a water miscible solvent or in the present of a phase transfer catalyst; or the halogenation is carried out in the mixture of sulfuric acid and an alkali metal salt of aqueous hydrogen halide . The process can be applied preferably on industrial scale.

### BACRGROUND OF THE INVENTION

2-Halogen-1-cyclopropyl-2-substituted phenylethanone compounds of general formula (III) are important starting compounds of tetrahydro thienopyridine derivatives, which are used in the pharmaceutical therapy. One of the most important representatives of tetrahydro thienopyridine derivatives is compound of formula (I), namely 5-[2-cyclopropyl-1-(2-fluorophenyl)-2-oxoethyl]-4,5,6,7-tetrahydrothieno[3,2-c]pyridin-2-yl acetate, having the international non-proprietary name prasugrel, used for the prevention and treatment of thrombosis and thromboembolism.

The platelet inhibitor prasugrel, its derivatives, and the process for their preparation was described for the first time by Hungarian Patent No. 218 785 and Hungarian Patent No. 211 876 and its equivalents. The object of the present invention relates to a process for the preparation of 2-halogen-1-cyclopropyl-2-substituted phenylethanone compounds of general formula (III), which are a very important structural part of the above mentioned compounds. The process of our invention is very well-applicable on an industrial scale and it enables the preparation of compounds of general formula (III) in high purity.

In the preparation process of prasugrel, and that of other effective platelet inhibitor compounds having a similar chemical structure, the key intermediates are 2-halogen-1-cyclopropyl-2-substituted phenylethanone compounds of general formula (III), which can have different substituents on the aromatic ring. The most important representative of these is compound of formula (IV), which is substituted by fluorine atom in position 2. From the literature only a few preparation processes are known for the synthesis of compounds of general formula (III) and these processes are narrowly applicable on industrial scale.

According to the preparation process described in Hungarian Patent No. 218 785 and Hungarian Patent No. 211 876, cyclopropyl compounds of general formula (III) are prepared by bromination of cyclopropyl benzyl ketone compounds of formula (II) in carbon tetrachloride, with an approximately equimolar amount of *N*-bromosuccinimide, in the presence of dibenzoyl peroxide under boiling for 8 hours. The yield of the process is 83 %, when a compound of general formula (III) is prepared, wherein R represents chlorine atom in position 2 and X represents bromine atom.

In United States Patent Application No. 2003/134872 a process is described for the preparation of compound of formula (IV) under similar conditions, wherein compound of formula (IV) is obtained by a purification using column chromatography and the yield of the reaction is 68 %.

The disadvantage of the above mentioned preparation processes is that N-bromosuccinimide is partly decomposed during the bromination, leading to the formation of elementary bromine. Bromine is a very corrosive molecule, therefore use of the bromine requires special conditions and special structural materials. Even the lowest iron contamination is able to increase the amount of the obtained by-products during the halogenation.

According to the preparation process described in United States Patent No. US 5,874,581 by the activation of compound of formula (II), wherein R represents a fluorine atom in position 2, compound of formula (III), wherein X is substituted by a chlorine atom, is obtained. The reaction is carried out in dichloromethane, at a temperature of 5 °C, with sulfuryl chloride or with chlorine gas. After preparation, the purity of the obtained crude product is 80% according to the GC measurements, it is used then for the preparation of prasugrel without purification. The disadvantage of the process is that sulfuryl chloride reacts very readily with water and the chlorine gas formed during the reaction is toxic and explosive. Both sulfuryl chloride and chlorine can be handled in chemical reactions only in a special apparatus and under very strict safety requirements.

The common disadvantage of the above mentioned processes are that halogenation is carried out in chlorinated hydrocarbons (in carbon tetrachloride or dichloromethane). These solvents, especially carbon tetrachloride, are very toxic solvents and their application on industrial scale is very rarely allowable.

Another disadvantage of the processes is that purification of the crude product is carried out by column chromatography. This purification process is not suitable for the preparation of a large quantity of the final product on industrial scale. A large amount of reagents is needed for the purification, therefore the process is more expensive and also disadvantageous for the environment.

The aim of the present invention is to avoid the disadvantages of the above processes and to develop an economical, simple preparation process with a good yield, which can be applied advantageously on an industrial scale. The further aim of the present invention to develop a preparation process, wherein the use of chlorinated solvents and other reagents, which are pollutive for the environment are avoided and to develop a process, which proceeds without purification by column chromatography.

The above mentioned aims are reached by the preparation process of the present invention.

### SUMMARY OF THE INVENTION

The object of the present invention is a process for the preparation of compounds of general formula (III), 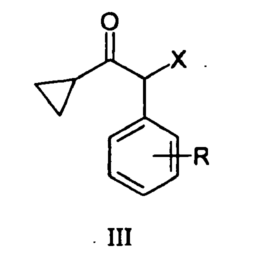 wherein R represents fluorine or chlorine atom, and X represents chlorine or bromine atom, from cyclopropyl-benzyl-ketone of general formula (II), 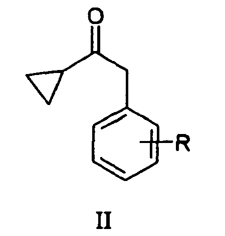 wherein R represents fluorine or chlorine atom, by halogenation. The halogenation is carried out in the mixture of aqueous hydrogen halide and aqueous hydrogen peroxide in the presence of a water miscible solvent or in the presence of a phase transfer catalyst; or in the mixture of sulfuric acid and an alkali metal salt of aqueous hydrogen halide and aqueous hydrogen peroxide.

The halogenation according to the present invention, wherein it is carried out in a mixture of aqueous hydrogen halide and aqueous hydrogen peroxide in the presence of 1-5 mol equivalents, preferably 2-4 mol equivalents of aqueous hydrogen halide and 1-10 mol equivalents, preferably 4-6 mol equivalents of aqueous hydrogen peroxide. In the reaction, aqueous hydrogen bromide solution or hydrogen bromide solution in acetic acid or hydrogen bromide gas, preferably 48 w/w% aqueous hydrogen bromide solution and aqueous hydrogen peroxide solution, preferably 25-40 w/w% aqueous hydrogen peroxide solution are used. The water miscible solution used in the halogenation, which is carried out in a mixture of aqueous hydrogen halide and aqueous hydrogen peroxide, is preferably dioxane, acetic acid, tetrahydrofuran or C₁₋₄ alcohol, having a straight or branched chain, preferably methyl alcohol, ethyl alcohol or isopropyl alcohol.

When the preparation process of the present invention is carried out without addition of a solvent, the applied phase transfer catalyst is quaternary ammonium salt, preferably benzyltriethyl ammonium chloride, tetrabutyl ammonium chloride, benzyl triethyl ammonium bromide or tetrabutyl ammonium bromide.

The reaction according to the present invention, wherein the halogenation of compound of general formula (III) is carried out in the mixture of sulfuric acid and an alkali metal salt of aqueous hydrogen halide, the alkali metal salt is preferably sodium bromide, sodium chloride, potassium bromide or potassium chloride. In the process preferably 1-5 mol equivalents of alkali metal salts and 2-10 mol equivalents of aqueous hydrogen peroxide solution are used.

The halogenation processes of the present invention are carried out at a temperature between 20 and 100 °C. At room temperature the reaction is completed within a few days and the purity of the obtained crude product is approximately 90 %, therefore it can be applied without further purification. At a temperature between 70 and 100 °C the reaction is completed within a few hours, but in this case, the crude product needs further purification, which can be easily carried out by distillation.

Another embodiment of the present invention is a process for the preparation of formula (I) 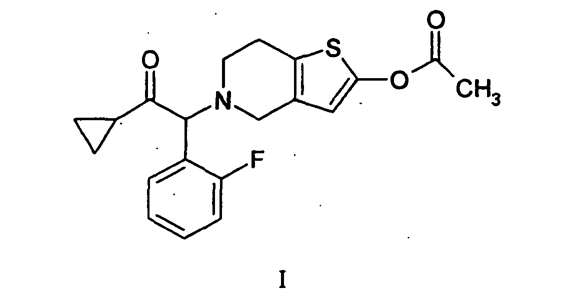 from compound of formula (III), 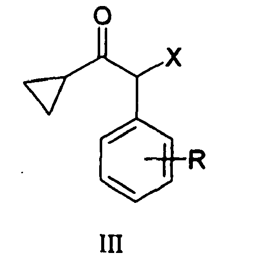 wherein R represents a fluorine atom in position 2, and X represents a chlorine or bromine atom wherein the cyclopropylbenzyl ketone compound of formula (II) 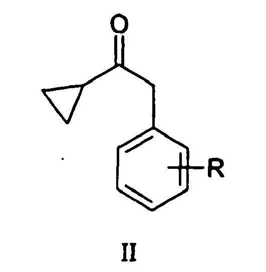 wherein R represents fluorine atom in position 2, is halogenated in a mixture of aqueous hydrogen halide and aqueous hydrogen peroxide in the present of a water miscible solvent or in the presence of a phase transfer catalyst; or the halogenation is carried out in a mixture of sulfuric acid and an alkali metal salt of aqueous hydrogen halide, thereafter the compound of formula (III), 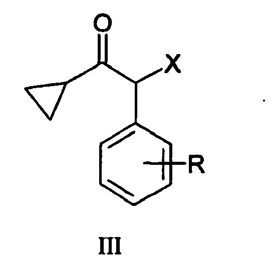 wherein R represents a fluorine atom in position 2, and X represents a chlorine or bromine atom is converted to compound of formula (I) 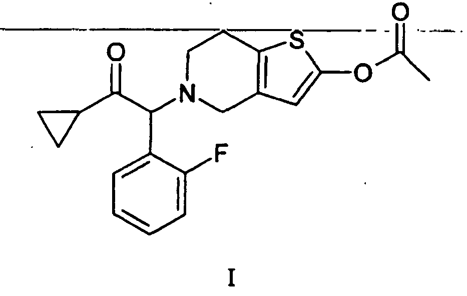 or an acid additional salt thereof according to the known methods.

### DETAILED DESCRIPTION OF THE INVENTION

The essence of our invention is that the halogenation is carried out without elementary halogen molecules, such as chlorine or bromine, or without the most often used halogenating agents known from the literature and prepared from halogens for example *N*-bromo-succinimide, bromodioxane, sulfuryl chloride etc.

A special advantage of our invention is that the use of environmentally dangerous halogenating agents are avoided and at the same time, it is not necessary to apply any special apparatus in the reaction, the process can be carried out in a commonly used apparatus. The solvent used the reaction is water and a water miscible solvent, for example acetic acid, dioxane, tetrahydrofuran or alcohols, having a short carbon chain. These water miscible solvents or a phase transfer catalyst ensure the dissolution of most of the compounds of formula (III) in the reaction mixture.

### Comparative experiments -

### Preparation of compound of formula (IV) from cyclopropyl-2-fluorobenzyl ketone:

A few halogenation reactions, well known from the literature, were carried out to prove the advantages of the claimed preparation process.

One of these reactions is a halogenation carried out with bromine, wherein the starting compound of the process is a compound of formula (II), wherein R represents a fluorine atom in position 2. Several reactions were carried out under different reaction conditions and the yields of the reactions and impurities were compared.

### 1. Preparation of compound of formula (IV) with bromine:

A compound of formula (II) was reacted with an equimolar amount of bromine in dichloromethane, at room temperature for 12 hours, until the colour of the bromine disappeared. According to the GC/MS measurement the reaction mixture contained

| | |
|---|---|
| 15 % | compound of formula (IV), |
| 35% | monobromo compound, derived from the opening of the cyclopropane ring, |
| 17% | dibromo compound, derived from the opening of the cyclopropane ring, and |
| 19% | unreacted starting compound. |

When pyridine was added to the reaction mixture, the obtained amount of compound of formula (IV) increased to 30 %, but the ratio of the compounds in the mixture was similar to the above described composition.

The same reaction was carried out in acetic acid. According to the GC/MS measurement, the reaction mixture contained only 3.5 % compound of formula (IV). The content of the mixture was the following:

| | |
|---|---|
| 3.5 % | compound of formula (IV), |
| 15% | monobromo compound, derived from the opening of the cyclopropane ring, |
| 47% | dibromo compound, derived from the opening of the cyclopropane ring, and |
| 31 % | unreacted starting compound. |

The conclusion of the above experiments is that compound of formula (IV) can not be prepared with bromine in a good yield.

### 2. Preparation of compound of formula (IV) with N-bromo-succinimide:

The process described in United States Patent Application No. 2003/134872 was reproduced, wherein the bromination was carried out with *N-*bromo-succinimide in carbon tetrachloride and the crude product was measured by GC/MS method. According to the GC/MS measurement the reaction mixture contained

| | |
|---|---|
| 68.5 % | compound of formula (IV), |
| 5.8% | monobromo compound, derived from the opening of the cyclopropane ring, |
| 5.5% | dibromo compound, derived from the opening of the cyclopropane ring, |
| 16.0 % | unreacted starting compound. |

The GC chromatogram of Figure I demonstrates the significant amount of impurities in the crude product.

The same reaction was carried out in dichloromethane, instead of carbon tetrachloride, and the reaction mixture was refluxed for 10 hours. The obtained amount of impurities was almost the same.

The efficient purification of compound of formula (IV) prepared by above reactions is practically impossible. Distillation is a process, which is suitable for the separation of compounds having significantly different boiling points. Among the products, obtained in the above reactions, compound of formula (IV) can be well separated from the starting compound and from the dibromo compound, because their molecular weights are significantly different. The monobromo impurities derived from the opening of the cyclopropane ring, having a total amount of 5.8 % can not be separated from compound of formula (IV), because their molecular weights are identical and their boiling points are also almost the same as that of the end-product. Moreover, these monobromo isomers react similarly to compound of formula (IV) in the following steps of the chemical reaction, therefore several by-products are formed in the next reaction step.

The purification of the reaction mixture by column chromatography is not successful either because the different impurities of the product are each others isomers, having very similar chemical structures, therefore their chromatographic characteristics are very close to each other.

### 3. Preparation of compound of formula (IV) according to the present invention:

Surprisingly it was found that by the preparation processes according to the present invention, compound of formula (IV) can be obtained in high purity compared to the methods described above (GC chromatogram of Figure II).

The bromination was carried out with a hydrogen peroxide - hydrogen bromide mixture in dioxane, at room temperature for a few days. According to the GC/MS measurement the reaction mixture contains

| | |
|---|---|
| 90.0 % | compound of formula (IV), |
| 0.1-0.2 % | monobromo compound, derived from the opening of the cyclopropane ring, |
| 1.3% | dibromo compound, derived from the opening of the cyclopropane ring, |
| 7.2 % | unreacted starting compound. |

The monobromo compounds having an opened cyclopropane ring were obtained only in an amount of a 0.1-0.2 %, the yield of the product was 90 % and the reaction mixture contained only 1.3% dibromo compound having an opened cyclopropane ring. The amount of the unreacted starting compound were 7.2 %.

Consequently the obtained crude product is contaminated by compounds that can be easily separated from the product by distillation. The starting compound of formula (II), as an impurity, does not have a halogen substituent in benzyl position, therefore it does not react in the following steps of the reaction, thus it does not cause contamination.

The quantitative measurements were carried out with an Agilent 6890N NetWork GC/MS System apparatus.

In the process of the invention, the bromination is carried out in dioxane. This solvent can be replaced by other water miscible solvents, for example with alcohols, e.g. ethanol, 2-propanol, or with acetic acid and ethers, e.g. tetrahydrofuran etc.

Compounds of general formula (II) cannot be dissolved in water, therefore it is advisable to add a water miscible organic solvent or a phase transfer catalyst to the reaction mixture to promote mixing as phase transfer catalyst, quaternary ammonium salts, preferably benzyltriethyl ammonium chloride, tetrabutyl ammonium chloride, benzyl triethyl ammonium bromide or tetrabutyl ammonium bromide can be used.

The applicability of our invention is further increased by the fact, that in the reaction, hydrogen halogenides (e.g.: hydrogen chloride, hydrogen bromide) can be substituted by alkali metal halogenides, for example by sodium bromide, potassium bromide, sodium chloride or potassium chloride. When alkali metal halogenides are used in the reaction, the reaction mixture should be acidified by the addition of e.g. sulfuric acid.

Surprisingly it was found that in the experiments carried out with alkali metal bromide (potassium bromide or sodium bromide) reagent, the amount of the obtained product is higher than 90 % and the dibromo contamination decreased to 2 %. In the reaction carried out with potassium bromide, the yield of the product was 94.0 %, that of the unreacted starting material was 3.2 %, besides 2.3 % of dibromo compounds, which was obtained by the opening of the cyclopropane ring.

Surprisingly it was also found that a purer crude product can be obtained in the preparation of the chloro derivative of compound of formula (III), wherein X represents chlorine atom and R represents fluorine atom in position 2, when alkali metal chloride derivatives are used in the halogenation instead of alkali metal bromide derivatives. In this case the monochloro derivative obtained by the ring opening of the cyclopropane ring does not contaminate the crude product and the dichloro contamination is under 2.0 %.

The above mentioned results are demonstrated by the following table, in Table I:

**Table I**

| **Reagents** | **Compound of formula (III)** | **Starting compound** | **Monobromo and monochloro compounds with opened ring** | **Dibromo and dichloro compounds with opened ring** |
|---|---|---|---|---|
| **reaction with *N*-bromsuccinimide** | 68.5% | 16.0 % | 5.8% | 5.5% |
| **hydrogen peroxide-hydrogen bromide system** | 90.0% | 7.2 % | 0.1-0.2 % | 1.3% |
| **hydrogen peroxide-hydrogen chloride system** | 95.3% | 3.7% | 0.1-0.2 % | 0.5% |
| **hydrogen peroxide-sulfuric acid - alkali metal bromide system** | 94.0% | 3.2% | 0.1-0.2 % | 2.3% |
| **hydrogen peroxide-sulfuric acid -alkali metal chloride system** | 93.2 % | 3.4% | 0.0% | 1.9% |

The product of the invention, the compounds of formula (III) are important starting compounds of the pharmaceutically applicable tetrahydro thienopyridine derivatives. Prasugrel, compound of formula (I) is prepared from compound of formula (III), wherein X represents chlorine atom or bromine atom and R represents fluorine atom in position 2, by bromination of compound of formula (II) according to one of the processes of the present invention, and subsequent reaction of the obtained bromo compound with 4,5,6,7-tetrahydro-thieno[2,3-c]pyridine of formula (V), according to the manufacturing process described in Hungarian Patent No. HU 211 876. In position 2 of the thiophene ring, the oxo group is obtained by the methods known from the art and prasugrel of formula (I) is finally obtained by O-acetylation under basic conditions and, if desired, it is converted to its acid addition salts.

### EXAMPLES

The invention is further elucidated by means of the following Examples without restricting the scope of the present invention to the Examples.

### Example 1

### 2-bromo-1-cyclopropyl-2-(2-fluorophenyl)-ethanone [compound of formula (IV)]

In a 1000 ml round-bottomed flask a solution of cyclopropyl-2-fluorobenzyl ketone (44,6 g, 0.25 mol), dioxane (500 ml), aqueous hydrogen peroxide solution (30 w/w%, 125 ml, 1.22 mol) and aqueous hydrogen bromide solution (48 w/w%, 71.3 ml, 0.63 mol) are added. The reaction mixture is warming to 50 °C and it is stirred for two hours at a temperature of 80-85 °C. To the colourless solution sodium sulfate (20 g) is added at 25 °C, it is stirred until dissolution, extracted and the organic layer is washed with aqueous sodium hydrogen carbonate (5 w/w %, 150 ml), separated and the organic layer is dried over magnesium sulfate and evaporated.

| | |
|---|---|
| The obtained product: | 59.2 g light yellow oil |
| Yield: | 82.9 % |
| Content (measured by GC): | 87.1 %. According to the results of the GC examination, it contains 7.5 % of the starting compound, 2.5 % of the monobromo impurity and 1.3 % of the dibromo derivatives. The product is purified by vacuum distillation. |
| Boiling point: | 90 °C/39.9966 Pa (0.3 Hgmm) |

The obtained product after distillation: 49.1 g colourless oil
Content of the obtained title product (measured by GC) after distillation: 97.5 %
IR (film): 3405, 3011, 1713, 1614, 1587, 1491, 1458, 1380, 1235, 1196, 1068, 1023.
¹H-NMR (CDCl₃, 500 MHz): 7.49 (t, 1H), 7.33 (m, 1H), 7.18 (t, 1H), 7.08 (t, 1H), 5.96 (s, 1H), 2.14 (m, 1H), 1.17 (m, 1H), 1.11 (m, 1H), 1.02 (m, 1H), 0.94 (m, 1H).
¹³C-NMR (CDCl₃, 125 MHz): 200.5, 159.7 (d, J = 249.0 Hz), 131.1 (d, J = 2.4 Hz), 130.8 (d, J = 8.3 Hz), 124.7 (d, J = 3.9 Hz), 123.4 (d, J = 13.2 Hz), 115.6 (d, J = 22.0 Hz), 48.3 (d, J = 2.9 Hz), 18.7, 12.7, 12.6.

### Example 2

### 2-bromo-1-cyclopropyl-2-(4-chlorophenyl)-ethanone [compound of formula (III) R=4-Cl]

In a 250 ml round-bottomed flask a solution of cyclopropyl-4-chlorobenzyl ketone (9.74 g, 50 mmol), dioxane (100 ml), aqueous hydrogen peroxide solution (30 w%, 25 ml, 0.23 mol) and aqueous hydrogen bromide solution (48 w%, 14.7 ml, 0.13 mol) are added. The reaction mixture is stirred for two hours at a temperature of 60-65 °C. To the colourless solution sodium sulphate (20 g) is added at 25 °C, it is stirred until dissolution, extracted and the upper organic layer is dried over magnesium sulphate and evaporated.

| | |
|---|---|
| The obtained product: | 15.6 g light yellow oil |
| Yield: | 87.2 % |
| Content (measured by GC): | 87.5 %. According to the results of the GC/MS examination, it contains 3.7 % starting compound and 0.5 % dibromo contamination. The product is purified by vacuum distillation. |
| Boiling point: | 116 °C / 39.9966 Pa (0.3 Hgmm) |
| The obtained product after distillation: | 11.0 g oil, which crystallises during standing. It is crystallised from n-hexane. |
| Melting point: | 38-40 °C white crystals, according to the GC measurements is 99.5 %. |

IR (KBr): 2978, 1699, 1492, 1415, 1381, 1074, 1016.
¹H-NMR (CDCl₃, 200 MHz): 7.40 (d, 2H), 7.35 (d, 2H), 5.56 (s, 1H), 2.15 (m, 1H), 1.12 (m, 2H), 1.00 (m, 2H).
¹³C-NMR (CDCl₃, 50 MHz): 201.3, 135.1, 134.0, 130.3, 129.1, 54.9, 18.7, 13.0, 12.9.

### Example 3

### 2-bromo-1-cyclopropyl-2-(2-fluorophenyl) ethanone [compound of formula (IV)]

In a 250 ml round-bottomed flask a solution of cyclopropyl-2-fluorbenzyl ketone (8.91 g, 50 mmol), acetic acid (50 ml), aqueous hydrogen peroxide solution (30 w%, 15 ml, 0.14 mol) and solution of hydrogen bromide in acetic acid (33 w%, 14.7 ml, 0.13 mol) are added. The reaction mixture is stirred for one hour at a temperature of 95 °C. The colourless solution is diluted with water (150 ml), extracted with toluene (100 ml) and the organic layer is separated, dried and evaporated in vacuo.

| | |
|---|---|
| The obtained product: | 11.4 g light yellow oil |
| Yield: | 75.4% |
| Content (GC/MS): | 85.0 %, it is contaminated with 5.2 % starting compound and 7.5 % dibromo derivative. The product is purified by vacuum distillation. |
| Boiling point: | 95 °C / 53.3288 Pa (0.4 Hgmm) |
| The obtained product after distillation: | 8.3 g colourless oil |

Content of the obtained title product (measured by GC) after distillation: 98.5 %

### Example 4

### 2-bromo-1-cyclopropyl-2-(2-fluorophenyl) ethanone [compound of formula (IV)]

In a 250 ml round-bottomed flask a solution of cyclopropyl-2-fluorbenzyl ketone (8.91 g, 50 mmol), ethanol (50 ml), aqueous hydrogen peroxide solution (30 w%, 30 ml, 0.29 mol) and aqueous hydrogen bromide solution (48 w%, 22.6 ml, 0.20 mol) are added. The reaction mixture is boiled for half an hour and the colourless solution is evaporated. To the residue water (50 ml) and ethyl acetate (50 ml) are added at 25 °C, the phases are separated and the organic phase is dried and evaporated.

| | |
|---|---|
| The obtained product: | 9.4 g light yellow oil |
| Yield: | 73.8% |
| Content (measured by GC/MS): | 83.4 % title product, which is contaminated with 15.2 % starting compound and 0.5 % dibromo derivative. |

### Example 5

### 2-bromo-1-cyclopropyl-2-(2-nuorophenyl)-etanon [compound of formula (IV)]

In a 250 ml round-bottomed flask a solution of cyclopropyl-2-fluorbenzyl ketone (8.91 g, 50 mmol), *N*-benzyl-triethyl-ammonium bromide (2.0 g), aqueous hydrogen peroxide solution (30 w%, 40 ml, 0.39 mol) and aqueous hydrogen bromide solution (48 w%, 28.3 ml, 0.25 mol) are added. The reaction mixture is stirred intensively for two hours at 85 °C and the product is extracted twice with ethyl acetate (2x50 ml) and the united phases of ethyl acetate are dried and evaporated.

| | |
|---|---|
| The obtained product: | 10.2 g light yellow oil |
| Yield: | 67.1 % |
| Content (measured by GC/MS): | 83.4 % title product, which is contaminated with 7.1 % starting compound and 7.5 % dibromo derivative. |

### Example 6

### 2-bromo-1-cyclopropyl-2-(2-fluorophenyl)-ethanone [compound of formula (IV)]

The preparation process is carried out according to example 1. with the difference that aqueous hydrogen bromide solution (48 w%, 71.3 ml, 0.63 mol) is added dropwise to the starting reaction mixture at 25 °C under cooling and under intensive stirring. The obtained mixture is stirred for 5 days at room temperature and the product is prepared according to example 1.

| | |
|---|---|
| The obtained product: | 59.2 g light yellow oil |
| Yield: | 82.9% |
| Content (measured by GC): | 90.0 %. According to the measurement by GC the crude product contains 7.2 % starting product and only 1.3 % dibromo derivative, which is less than the obtained bromo derivative in example 1. The product, if it is necessary, can be purified by distillation. |
| Boiling point: | 90 °C / 39.9966 Pa (0.3 Hgmm) |
| The obtained product after distillation: | 49.1 g colourless oil |
| Content of (GC) after distillation: | 97.5 % |

### Example 7

### 2-chloro-1-cyclopropyl-2-(2-fluorophenyl)-ethanone [compound of formula (III) X=Cl, R=2-F)]

The preparation process is carried out according to example 1. with the difference that instead of hydrogen bromide, concentrated aqueous solution of hydrochloric acid (105 ml, 1.24 mol) is added to the reaction mixture. This solution is added dropwise to the starting reaction mixture at 25 °C under cooling and under intensive stirring. The obtained mixture is stirred for 3 days at room temperature and the product is processed according to example 1.

| | |
|---|---|
| The obtained product: | 51.0 g colourless oil |
| Yield: | 82.9 %. |
| Content (measured by GC): | 94.5 %. According to the measurement by GC/MS the crude product contains 3.5 % starting product and 1.4 % dichloro derivative, therefore a further purification is not necessary. |

### Example 8

### 2-chloro-1-cyclopropyl-2-(2-fluorophenyl)-ethanone [compound of formula (III) X=Cl, R=2-F)]

In a 250 ml round-bottomed flask a solution of cyclopropyl-2-fluorbenzyl ketone (9.74 g, 50 mmol), dioxane (100 ml), aqueous hydrogen peroxide solution (30 w%, 25 ml, 0.23 mol) and concentrated aqueous solution of hydrochloric acid (11.0 ml, 0.13 mol) are added. The reaction mixture is stirred for two hours at 80 °C, then it is cooled and to the colourless solution sodium sulphate (20 g) and ethyl acetate (50 ml) is added to the reaction mixture. The organic upper layer is extracted with aqueous sodium hydrogen carbonate; it is dried over magnesium sulphate and evaporated.

| | |
|---|---|
| The obtained product: | 10.3 g colourless oil |
| Yield: | 86.1 % |
| Content (measured by GC): | 95.3 %. According to the measurement by GC the product contains 3.7 % starting product and 0.5 % dichloro contamination. The crude product, if it is necessary, can be purified by vacuum distillation. |
| Boiling point: | 85 °C / 26.6644 Pa (0.2 Hgmm) |
| The obtained product after distillation: | 8.2 g colourless oil |
| Content (measured by GC) after distillation: | 98.5 %. |

### Example 9

### 2-bromo-1-cyclopropyl-2-(2-fluorophenyl)-ethanone [compound of formula (IV)]

The preparation process is carried out according to example 1. with the difference that instead of aqueous hydrogen bromide solution (48 w%, 71.3 ml, 0.63 mol), potassium bromide (75.0 g, 0.63 mol) and slowly sulfuric acid (30.0 g, 0.30 mol) are added to the starting reaction mixture. The title product is prepared according to example 1.

| | |
|---|---|
| The obtained product: | 48.8 g light yellow oil |
| Yield: | 86.3 % |
| Content (measured by GC): | 94.0 %. According to the measurement by GC the product contains 3.2 % starting product and 2.3 % dibromo derivative. |

### Example 10

### 2-bromo-1-cyclopropyl-2-(2-fluorophenyl)-ethanone [compound of formula (IV)]

The preparation process is carried out according to example 1. with the difference that instead of aqueous hydrogen bromide solution (48 w%, 71.3 ml, 0.63 mol), under cooling potassium bromide (52.0 g, 0.50 mol) at 25 °C and and slowly sulfuric acid (30.0 g, 0.30 mol) are added to the starting reaction mixture. The reaction mixture is stirred for 3 days at room temperature and the title product is processed according to example 1.

| | |
|---|---|
| The obtained product: | 48.5 g light yellow oil |
| Yield: | 84.1% |
| Content (measured by GC): | 92.1 %. According to the measurement by GC the crude product contains 4.3 % starting product and 1.5 % dibromo derivative. The title product, if it is necessary, can be purified by distillation. |

### Example 11

### 2-chloro-1-cyclopropyl-2-(2-fluorophenyl)-ethanone [compound of formula (III) X=Cl, R=2-F)]

The process is carried out according to example 1. with the difference that instead of aqueous hydrogen bromide solution (48 w%, 71.3 ml, 0.63 mol), under cooling sodium chloride (29.5 g, 0.50 mol) at 25 °C and and slowly sulfuric acid (25.0 g, 0.25 mol) are added to the starting reaction mixture. The reaction mixture is stirred for 48 hours at room temperature and the title product is processed according to example 1.

| | |
|---|---|
| The obtained product: | 60.4 g light yellow oil |
| Content (measured by GC): | 93.2 %. According to the measurement by GC the crude product contains 3.4 % starting product and 1.9 % dichloro derivative. The title product, if it is necessary, can be purified by distillation. |

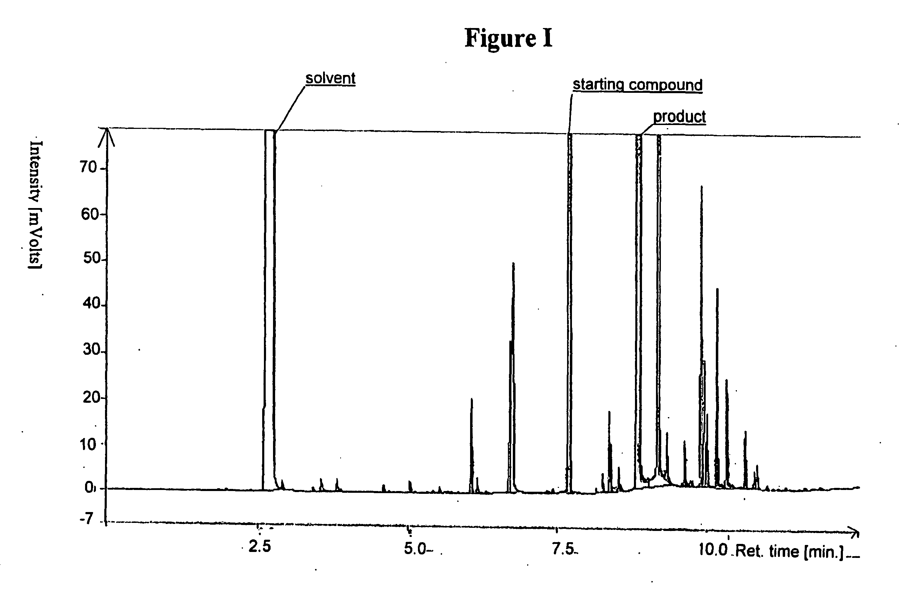

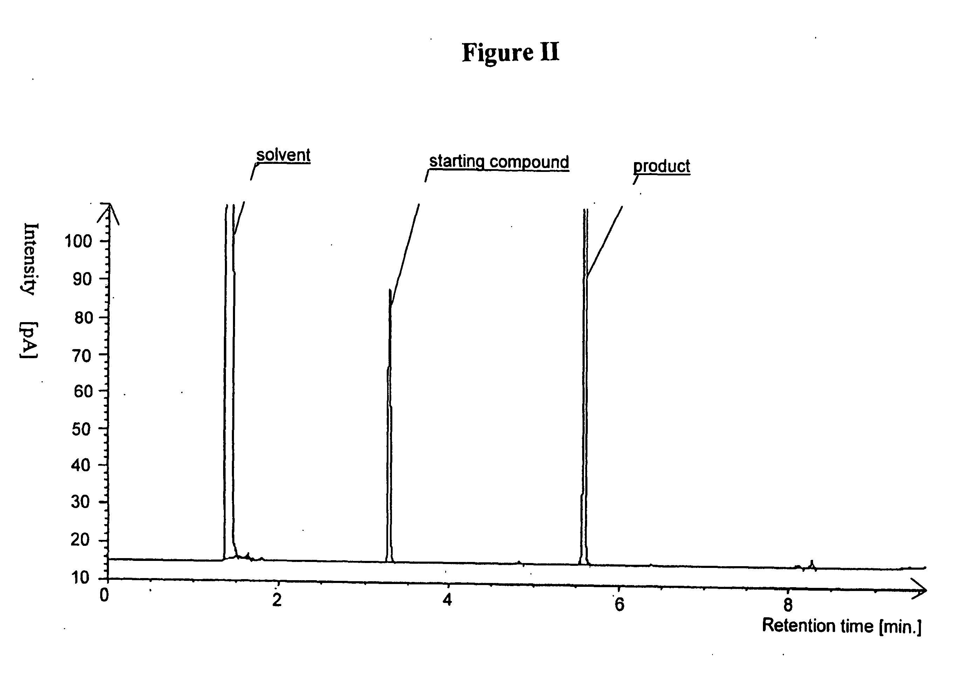

## Claims

1. Process for the preparation of compounds of general formula (III), 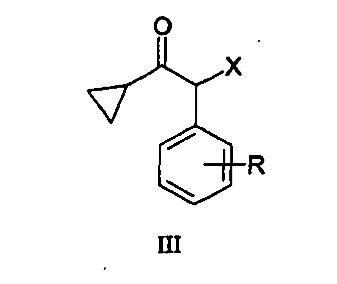 wherein R represents fluorine or chlorine atom and X represents chlorine or bromine atom, by halogenation of cyclopropyl benzyl ketone of general formula (II), 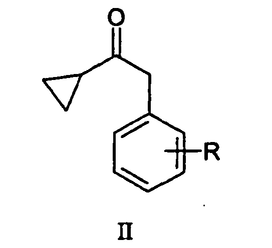 wherein R represents fluorine or chlorine atom, **characterized in that**
a. the halogenation is carried out in a mixture of aqueous hydrogen halide and aqueous hydrogen peroxide in the present of a water miscible solvent or in the present of a phase transfer catalyst; or
b. the halogenation is carried out in a mixture of an alkali metal salt of aqueous hydrogen halide, aqueous hydrogen peroxide and sulfuric acid.

2. Process according to claim 1, **characterized in that** the reaction is carried out in the presence of 1-5 mol equivalents of aqueous hydrogen halide and 1-10 mol equivalents of aqueous hydrogen peroxide, calculated for the compound of formula (II).

3. Process according to claim 1, **characterized in that** the reaction is carried out preferably in the presence of 2-4 mol equivalents aqueous hydrogen halide and 4-6 mol equivalents aqueous hydrogen peroxide calculated for the compound of formula (II).

4. Process according to claim 1, **characterized in that** aqueous hydrogen bromide solution or hydrogen bromide solution in acetic acid or hydrogen bromide gas, preferably 48 w% aqueous hydrogen bromide solution is used in the reaction.

5. Process according to claim 1, **characterized in that** aqueous hydrogen peroxide solution, preferably 25-40 w% aqueous hydrogen peroxide solution is used in the reaction.

6. Process according to claim 1, **characterized in that** the water miscible solvent is dioxane, acetic acid, tetrahydrofuran or C₁₋₄ alcohol, having a straight or branched chain, preferably methyl alcohol, ethyl alcohol or isopropyl alcohol.

7. Process according to claim 1, **characterized in that** the phase transfer catalyst is quaternary ammonium salt, preferably benzyltriethyl ammonium chloride, tetrabutyl ammonium chloride, benzyl triethyl ammonium bromide or tetrabutyl ammonium bromide.

8. Process according to claim 1, **characterized in that** the alkali metal salt is sodium bromide, sodium chloride, potassium bromide or potassium chloride.

9. Process according to claim 1, **characterized in that** 1-5 mol equivalents of alkali metal salts and 2-10 mol equivalent of aqueous hydrogen peroxide solution are used in the reaction.

10. Process for the preparation of formula (I) 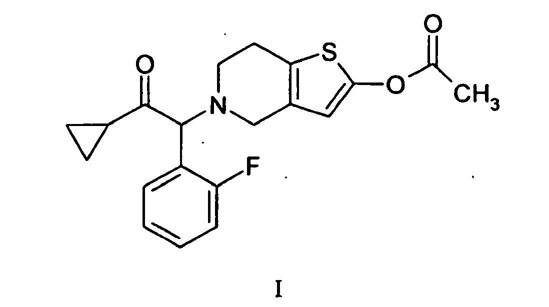 from compound of formula (III), 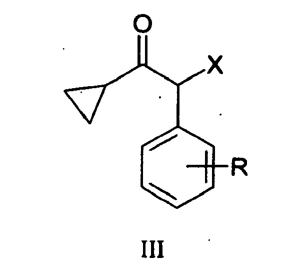 wherein R represents a fluorine atom in position 2, and X represents a chlorine or bromine atom **characterized in that** the cyclopropyl benzyl ketone compound of formula (II) 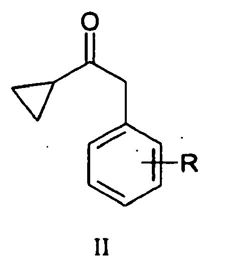 wherein R represents fluorine atom in position 2, is halogenated in a mixture of aqueous hydrogen halide and aqueous hydrogen peroxide in the presence of a water miscible solvent or in the presence of a phase transfer catalyst; or the halogenation is carried out in a mixture of sulfuric acid and an alkali metal salt of aqueous hydrogen halide, and the obtained compound of formula (III), 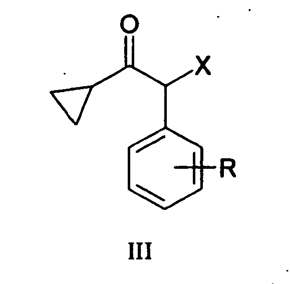 wherein R represents a fluorine atom in position 2, and X represents a chlorine or bromine atom is then converted to compound of formula (I) 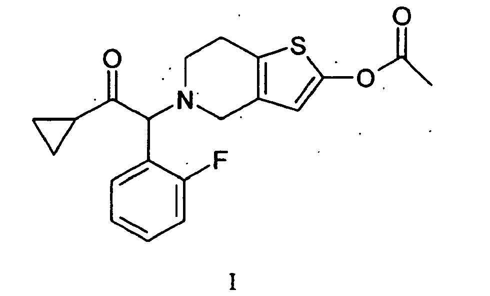 or an acid addition salt thereof according to the known methods.

## Patentansprüche

1. Verfahren für die Herstellung von Verbindungen der allgemeinen Formel (III): 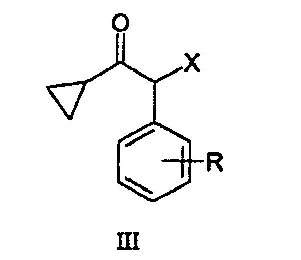 worin R für ein Fluor- oder Chloratom steht und X für ein Chlor- oder Bromatom steht, durch Halogenierung von Cyclopropylbenzylketon der allgemeinen Formel (II): 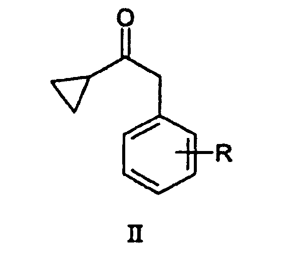 worin R für ein Fluor- oder Chloratom steht, **dadurch gekennzeichnet, dass**
a. die Halogenierung in einer Mischung von wässrigem Halogenwasserstoff und wässrigem Wasserstoffperoxid in Gegenwart eines wassermischbaren Lösungsmittels oder in Gegenwart eines Phasenübergangskatalysators durchgeführt wird; oder
b. die Halogenierung in einer Mischung eines Alkalimetallsalzes von wässrigem Halogenwasserstoff, wässrigem Wasserstoffperoxid und Schwefelsäure durchgeführt wird.

2. Verfahren gemäß Anspruch 1, **dadurch gekennzeichnet, dass** die Reaktion in Gegenwart von 1-5 Moläquivalenten an wässrigem Halogenwasserstoff und 1-10 Moläquivalenten an wässrigem Wasserstoffperoxid, berechnet für die Verbindung der Formel (II), durchgeführt wird.

3. Verfahren gemäß Anspruch 1, **dadurch gekennzeichnet, dass** die Reaktion vorzugsweise in Gegenwart von 2-4 Moläquivalenten an wässrigem Halogenwasserstoff und 4-6 Moläquivalenten an wässrigem Wasserstoffperoxid, berechnet für die Verbindung der Formel (II), durchgeführt wird.

4. Verfahren gemäß Anspruch 1, **dadurch gekennzeichnet, dass** wässrige Bromwasserstofflösung oder Bromwasserstofflösung in Essigsäure oder Bromwasserstoffgas, vorzugsweise 48 Gew.-%ige wässrige Bromwasserstofflösung, in der Reaktion verwendet werden.

5. Verfahren gemäß Anspruch 1, **dadurch gekennzeichnet, dass** wässrige Wasserstoffperoxidlösung, vorzugsweise 25 - 40 Gew.-%ige wässrige Wasserstoffperoxidlösung, in der Reaktion verwendet werden.

6. Verfahren gemäß Anspruch 1, **dadurch gekennzeichnet, dass** das wassermischbare Lösungsmittel Dioxan, Essigsäure, Tetrahydrofuran oder C₁₋₄-Alkohol mit einer geraden oder verzweigten Kette, vorzugsweise Methylalkohol, Ethylalkohol oder Isopropylalkohol, ist.

7. Verfahren gemäß Anspruch 1, **dadurch gekennzeichnet, dass** der Phasenübergangskatalysator quaternäres Ammoniumsalz, vorzugsweise Benzyltriethylammoniumchlorid, Tetrabutylammoniumchlorid, Benzyltriethylammoniumbromid oder Tetrabutylammoniumbromid, ist.

8. Verfahren gemäß Anspruch 1, **dadurch gekennzeichnet, dass** das Alkalimetallsalz Natriumbromid, Natriumchlorid, Kaliumbromid oder Kaliumchlorid ist.

9. Verfahren gemäß Anspruch 1, **dadurch gekennzeichnet, dass** 1-5 Moläquivalente an Alkalimetallsalzen und 2-10 Moläquivalente an wässriger Wasserstoffperoxidlösung in der Reaktion verwendet werden.

10. Verfahren für die Herstellung der Formel (I): 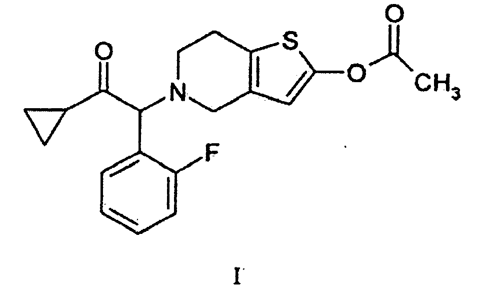 aus der Verbindung der Formel (III): 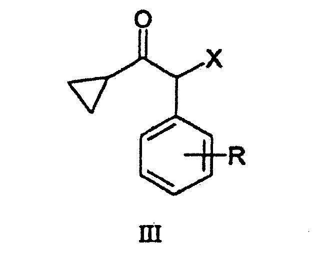 worin R für ein Fluoratom an der Position 2 steht und X für ein Chlor- oder Bromatom steht, **dadurch gekennzeichnet, dass** die Cyclopropylbenzylketonverbindung der Formel (II): 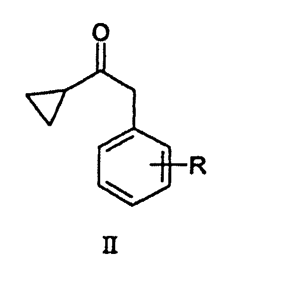 worin R für ein Fluoratom an der Position 2 steht, in einer Mischung von wässrigem Halogenwasserstoff und wässrigem Wasserstoffperoxid in Gegenwart eines wassermischbaren Lösungsmittels oder in Gegenwart eines Phasenübergangskatalysators durchgeführt wird; oder die Halogenierung in einer Mischung von Schwefelsäure und einem Alkalimetallsalz von wässrigem Halogenwasserstoff durchgeführt wird und die erhaltene Verbindung der Formel (III): 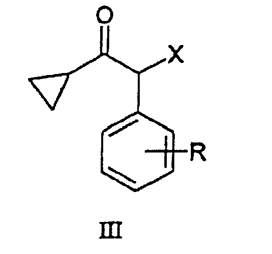 worin R für ein Fluoratom an der Position 2 steht und X für ein Chlor- oder Bromatom steht, danach zu der Verbindung der Formel (I): 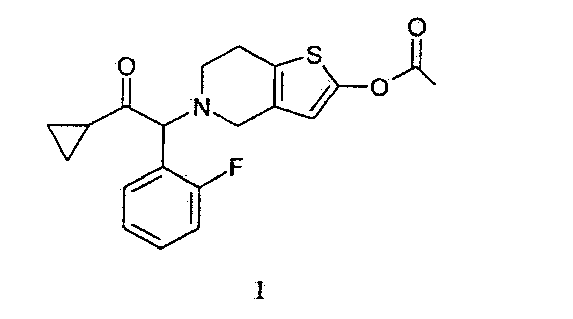 oder einem Säureadditionssalz davon gemäß den bekannten Verfahren umgewandelt wird.

## Revendications

1. Procédé pour la préparation de composés de formule générale (III), 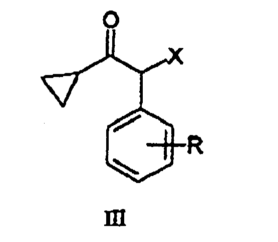 dans laquelle R représente un atome de fluor ou de chlore et X représente un atome de chlore ou de brome, par halogénation de cyclopropyl benzyl cétone de formule générale (II), 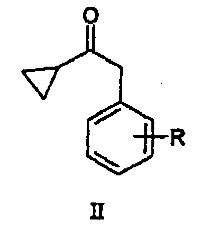 dans laquelle R représente un atome de fluor ou de chlore, **caractérisé en ce que**
a. l'halogénation est effectuée dans un mélange d'halogénure d'hydrogène aqueux et de peroxyde d'hydrogène aqueux en présence d'un solvant miscible à l'eau ou en présence d'un catalyseur de transfert de phase ; ou
b. l'halogénation est effectuée dans un mélange d'un sel de métal alcalin d'halogénure d'hydrogène aqueux, de peroxyde d'hydrogène aqueux et d'acide sulfurique.

2. Procédé selon la revendication 1, **caractérisé en ce que** la réaction est effectuée en présence de 1 à 5 équivalents molaires d'halogénure d'hydrogène aqueux et de 1 à 10 équivalents molaires de peroxyde d'hydrogène aqueux, calculés pour le composé de formule (II).

3. Procédé selon la revendication 1, **caractérisé en ce que** la réaction est effectuée de préférence en présence de 2 à 4 équivalents molaires d'halogénure d'hydrogène aqueux et de 4 à 6 équivalents molaires de peroxyde d'hydrogène aqueux calculés pour le composé de formule (II).

4. Procédé selon la revendication 1, **caractérisé en ce qu'**on utilise une solution de bromure d'hydrogène aqueux ou une solution de bromure d'hydrogène dans de l'acide acétique ou du bromure d'hydrogène gazeux, de préférence une solution de bromure d'hydrogène aqueux à 48 % en poids, dans la réaction.

5. Procédé selon la revendication 1, **caractérisé en ce qu'**une solution de peroxyde d'hydrogène aqueux, de préférence une solution de peroxyde d'hydrogène aqueux à 25-40 % en poids, est utilisée dans la réaction.

6. Procédé selon la revendication 1, **caractérisé en ce que** le solvant miscible à l'eau est du dioxane, de l'acide acétique, du tétrahydrofuranne ou un alcool en C₁₋₄, ayant une chaîne linéaire ou ramifiée, de préférence l'alcool méthylique, l'alcool éthylique ou l'alcool isopropylique.

7. Procédé selon la revendication 1, **caractérisé en ce que** le catalyseur de transfert de phase est un sel d'ammonium quaternaire, de préférence le chlorure de benzyltriéthyl ammonium, le chlorure de tétrabutyl ammonium, le bromure de benzyl triéthyl ammonium ou le bromure de tétrabutyl ammonium.

8. Procédé selon la revendication 1, **caractérisé en ce que** le sel de métal alcalin est le bromure de sodium, le chlorure de sodium, le bromure de potassium ou le chlorure de potassium.

9. Procédé selon la revendication 1, **caractérisé en ce que** 1 à 5 équivalents molaires de sels de métal alcalin et 2 à 10 équivalents molaires de solution de peroxyde d'hydrogène aqueux sont utilisés dans la réaction.

10. Procédé pour la préparation de formule (I) 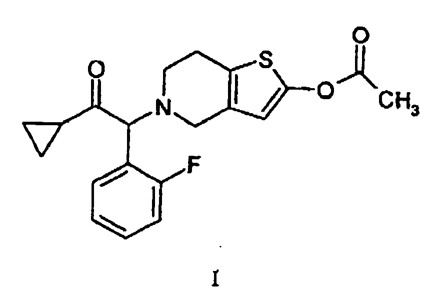 à partir du composé de formule (III), 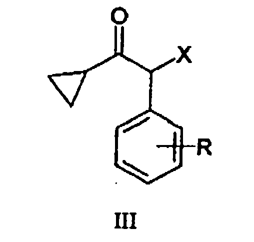 dans laquelle R représente un atome de fluor en position 2, et X représente un atome de chlore ou de brome, **caractérisé en ce que** le composé de cyclopropyl benzyl cétone de formule (II) 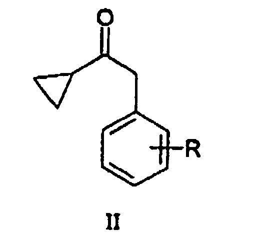 dans laquelle R représente un atome de fluor en position 2, est halogéné dans un mélange d'halogénure d'hydrogène aqueux et de peroxyde d'hydrogène aqueux en présence d'un solvant miscible à l'eau ou en présence d'un catalyseur de transfert de phase ; ou l'halogénation est effectuée dans un mélange d'acide sulfurique et d'un sel de métal alcalin d'halogénure d'hydrogène aqueux, et le composé obtenu de formule (III), 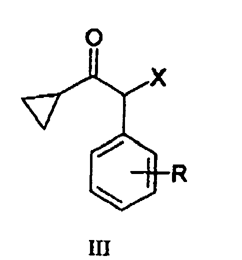 dans laquelle R représente un atome de fluor en position 2, et X représente un atome de chlore ou de brome, est ensuite converti en un composé de formule (I) 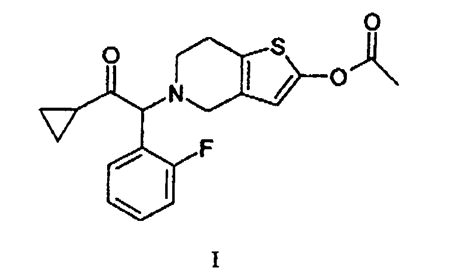 ou un sel d'addition de celui-ci avec un acide d'après les procédés connus.
